# EUROPÄISCHE PATENTANMELDUNG

(11) **EP 2 796 445 A1**
(43) Veröffentlichungstag der Anmeldung: **29.10.2014**
(21) Anmeldenummer: 14001820.1
(22) Anmeldetag: 18.03.2009
(51) Int. Cl.: C07C 231/02, C07C 235/06

(54) **Kontinuierliches Verfahren zur Herstellung von Amiden aliphatischer Hydroxycarbonsäuren**

(30) Priorität: 04.04.2008 DE 102008017213
(62) Teilanmeldung aus: 09728707.2
(71) Anmelder: Clariant Finance (BVI) Limited, Road Town, Tortola (VG)
(72) Erfinder: Krull, Matthias, 55296 Harxheim (DE); Morschhäuser, Roman, 55122 Mainz (DE); Hess, Joachim, 65719 Hofheim (DE); Milbradt, Robert, 65191 Wiesbaden (DE); Scherl, Franz-Xaver, 84508 Burgkirchen (DE); Wacker, Andreas, 8163 Mannheim (DE); Seebach, Michael, 65719 Hofheim (DE); Appel, Jörg, 84577 Tüssling (DE)
(74) Vertreter: Jacobi, Carola

(57) **Zusammenfassung**

Gegenstand der Erfindung ist ein kontinuierliches Verfahren zur Herstellung von Hydroxycarbonsäureamiden, indem mindestens eine Hydroxycarbonsäure der Formel I

HO-R³-COOH (I)

worin R³ für einen gegebenenfalls substituierten aliphatischen Kohlenwasserstoffrest mit 1 bis 100 Kohlenstoffatomen steht,
mit mindestens einem Amin der Formel II

HNR¹R² (II)

worin R¹ und R² unabhängig voneinander für Wasserstoff oder einen Kohlenwasserstoffrest mit 1 bis 100 C-Atomen stehen,
zu einem Ammoniumsalz umgesetzt wird und dieses Ammoniumsalz nachfolgend unter Mikrowellenbestrahlung in einem Reaktionsrohr, dessen Längsachse sich in der Ausbreitungsrichtung der Mikrowellen eines Monomode-Mikrowellenapplikators befindet, zum Hydroxycarbonsäureamid umgesetzt wird.

## Beschreibung

Amide aliphatischer Hydroxycarbonsäuren sind als chemische Rohstoffe sehr interessant. Von niederen Hydroxycarbonsäuren abgeleitete Amide sind Flüssigkeiten mit sehr hohem Lösevermögen und speziell tertiäre Amide haben als aprotische, polare Lösemittel hervorragende Eigenschaften. Insbesondere tertiäre Amide chiraler Hyrdoxycarbonsäuren eignen sich als Hilfsmittel bei der Enantiomerentrennung. Amide längerkettiger Hydroxycarbonsäuren haben interessante Eigenschaften als oberflächenaktive Substanzen.

Bei der technischen Herstellung wird üblicherweise ein reaktives Derivat einer Hydroxycarbonsäure wie Säureanhydrid, Säurechlorid oder Ester mit einem Amin umgesetzt. Dies führt einerseits zu hohen Herstellkosten und andererseits zu unerwünschten Begleitprodukten wie beispielsweise Salzen oder Säuren, die abgetrennt und entsorgt oder aufgearbeitet werden müssen. So entstehen beispielsweise in der Schotten-Baumann-Synthese, nach der zahlreiche Hydroxycarbonsäureamide großtechnisch hergestellt werden, equimolare Mengen Kochsalz. Die erstrebenswerte direkte thermische Kondensation von Carbonsäuren und Aminen erfordert sehr höhe Temperaturen und lange Reaktionszeiten, wobei jedoch nur moderate Ausbeuten erhalten werden (J. Am. Chem. Soc., 59 (1937), 401-402). Weiterhin ist die Auftrennung von eingesetzter Säure und gebildetem Amid oftmals äußerst aufwendig, da beide häufig sehr ähnliche Siedepunkte besitzen und zudem Azeotrope bilden.

GB-414 366 offenbart ein Verfahren zur Herstellung von substituierten Amiden durch thermische Kondensation. In den Beispielen werden höher siedende Carbonsäuren mit gasförmigen sekundären Aminen bei Temperaturen von 200 - 250 °C umgesetzt. Die Rohprodukte werden mittels Destillation oder Bleichen aufgereinigt.

Problematisch bei diesen Herstellverfahren sind insbesondere sehr lange Reaktionszeiten zur Erzielung eines kommerziell interessanten Umsatzes sowie die Korrosivität der Reaktionsgemische aus Säure, Amin, Amid und Reaktionswasser, die bei den erforderlichen hohen Reaktionstemperaturen metallische Reaktionsgefäße stark angreifen bzw. auflösen. Die dadurch in die Produkte eingetragenen Metallgehalte sind sehr unerwünscht, da sie die Produkteigenschaften nicht nur hinsichtlich ihrer Farbe beeinträchtigen sondern auch Zersetzungsreaktionen katalysieren und damit die Ausbeute reduzieren. Umgangen werden kann letzteres Problem zum Teil durch spezielle Reaktionsgefäße aus hoch korrosionsbeständigen Materialien oder mit entsprechenden Beschichtungen, was aber trotzdem lange Reaktionszeiten erfordert und somit zu in ihrer Farbe beeinträchtigten Produkten führt. Als unerwünschte Nebenreaktionen sind beispielsweise eine Oxidation des Amins, eine thermische Disproportionierung sekundärer Amine zu primärem und tertiärem Amin und eine Decarboxylierung der Carbonsäure zu nennen. Weiterhin werden bei thermischen Kondensationen von Hydroxycarbonsäuren mit Aminen eine Eliminierung der Hydroxylgruppe unter Bildung von Olefinen, eine Aminolyse der Hydroxylgruppe unter Bildung von Amidaminen wie auch Veresterungen unter Bildung von Oligomeren und Polymeren als Nebenreaktionen beobachtet. Durch alle diese Nebenreaktionen wird die Ausbeute an Zielprodukt herabgesetzt.

Ein neuerer Ansatz zur Synthese von Amiden ist die durch Mikrowellen unterstützte Umsetzung von Carbonsäuren und Aminen zu Amiden.

Vázquez-Tato, Synlett., 1993, 506, offenbart die Verwendung von Mikrowellen als Heizquelle für die Herstellung von Amiden aus Carbonsäuren und arylaliphatischen Aminen über die Ammoniumsalze. Die Synthesen erfolgten im 10 mmol-Maßstab.

Gelens et al., Tetrahedron Letters 2005, 46(21), 3751-3754, offenbart eine Vielzahl an Amiden, die unter Zuhilfenahme von Mikrowellenstrahlung synthetisiert wurden, jedoch keine Hydroxycarbonsäureamide. Die Synthesen erfolgten in 10 ml-Gefäßen.

US-6017426 lehrt ein Verfahren zur Herstellung von Ceramiden, indem ein Aminoalkohol mit einer Carbonsäure zur Reaktion gebracht wird. Die Reaktion wird bei Temperaturen von bis zu 180 °C unter Einfluss von Mikrowellenbestrahlung durchgeführt.

R. Plantier-Royon et al., C.R. Chimie 7 (2004), Seiten 119 - 123, lehrt ein Verfahren zur Herstellung von aliphatischen, aromatischen oder funktionalisierten Amiden der Weinsäure aus Weinsäure und Aminen unter Mikrowellenbestrahlung.

Massicot et al., Synthesis 2001, Nr. 16, S. 2441-2444 offenbart die Synthese von Tartramiden unter dem Einfluss von Mikrowellen, wobei wirtschaftliche Erwägungen und Vorteile im Hinblick auf Sicherheit und Umwelt für den Einsatz von Mikrowellen herausgestellt werden; die Reaktionen werden jedoch nur im mmol-Maßstab durchgeführt. Umsetzungen mit sekundären Aminen konnten dabei nicht beobachtet werden.

Das Scale-Up derartiger mikrowellenunterstützter Reaktionen aus dem Labor in einen technischen Maßstab und damit die Entwicklung von Anlagen, die für eine Produktion von mehreren Tonnen wie beispielsweise mehreren zehn, mehreren hundert oder mehreren tausend Tonnen pro Jahr mit für großtechnische Anwendungen interessanten Raum-Zeit-Ausbeuten geeignet sind, konnte bisher jedoch nicht realisiert werden. Ursache dafür ist zum einen die üblicherweise auf einige Millimeter bis wenige Zentimeter begrenzte Eindringtiefe von Mikrowellen in das Reaktionsgut, was insbesondere in Batch-Verfahren durchgeführte Reaktionen auf kleine Gefäße beschränkt oder in gerührten Reaktoren zu sehr langen Reaktionszeiten führt. Einer für die Bestrahlung großer Substanzmengen mit Mikrowellen wünschenswerten Erhöhung der Feldstärke sind insbesondere in den bisher bevorzugt zum Scale-Up chemischer Reaktionen eingesetzten Multimode-Geräten durch dann auftretende Entladungsvorgänge und Plasmabildung enge Grenzen gesetzt. Weiterhin bereitet die zu lokalen Überhitzungen des Reaktionsgutes führende, durch mehr oder weniger unkontrollierte Reflektionen der in den Mikrowellenofen eingestrahlten Mikrowellen an dessen Wänden und dem Reaktionsgut verursachte Inhomogenität des Mikrowellenfeldes in den üblicherweise eingesetzten Multimode-Mikrowellengeräten Probleme bei der Maßstabsvergrößerung. Zudem bereitet der sich während der Reaktion oftmals ändernde Mikrowellen-Absorptionskoeffizient des Reaktionsgemischs Schwierigkeiten hinsichtlich einer sicheren und reproduzierbaren Reaktionsführung.

C. Chen et al., J. Chem. Soc., Chem. Commun., 1990, 807 - 809, beschreiben einen kontinuierlichen Labor-Mikrowellenreaktor, bei dem das Reaktionsgut durch eine in einem Mikrowellenofen montierte Teflon-Rohrschlange geführt wird. Ein ähnlicher kontinuierlicher Labor-Mikrowellenreaktor wird von Cablewski et al., J. Org. Chem. 1994, 59, 3408-3412 zur Durchführung verschiedenster chemischer Reaktionen beschrieben. In beiden Fällen erlaubt die im Multimode betriebene Mikrowelle jedoch kein Up-Scaling in den großtechnischen Bereich. Ihr Wirkungsgrad bezüglich der Mikrowellenabsorption des Reaktionsguts ist auf Grund der in Multimode-Mikrowellenapplikatoren auf den Applikatorraum mehr oder weniger homogen verteilten und nicht auf die Rohrschlange fokussierten Mikrowellenenergie niedrig. Eine starke Erhöhung der eingestrahlten Mikrowellenleistung führt hier zu unerwünschten Plasma-Entladungen. Weiterhin machen die als Hot-Spots bezeichneten, sich zeitlich verändernden räumlichen Inhomogenitäten des Mikrowellenfeldes eine sichere und reproduzierbare Reaktionsführung im großen Maßstab unmöglich.

Weiterhin sind Monomode- bzw. Singlemode-Mikrowellenapplikatoren bekannt, bei denen mit einem einzigen Wellen-Modus gearbeitet wird, der sich in nur einer Raumrichtung ausbreitet und durch exakt dimensionierte Wellenleiter auf das Reaktionsgefäß fokussiert wird. Diese Geräte erlauben zwar höhere lokale Feldstärken, sind bisher aber auf Grund der geometrischen Anforderungen (z. B. ist die Intensität des elektrischen Feldes an seinen Wellenbergen am größten und geht an den Knotenpunkten gegen Null) bisher auf kleine Reaktionsvolumina (≤ 50 ml) im Labormaßstab beschränkt.

Es wurde daher ein Verfahren zur Herstellung von Hydroxycarbonsäureamiden gesucht, bei dem Hydroxycarbonsäure und Amin auch im technischen Maßstab unter Mikrowellenbestrahlung zum Amid umgesetzt werden können. Ein besonderes Interesse bestand dabei an tertiären Amiden von Hydroxycarbonsäuren. Dabei sollen möglichst hohe, das heißt bis zu quantitative Umsetzungsraten und Ausbeuten erzielt werden. Das Verfahren soll weiterhin eine möglichst energiesparende Herstellung der Carbonsäureamide ermöglichen, das heißt, die eingesetzte Mikrowellenleistung soll möglichst quantitativ vom Reaktionsgut absorbiert werden und das Verfahren somit einen hohen energetischen Wirkungsgrad bieten. Dabei sollen keine bzw. nur untergeordnete Mengen an Nebenprodukten anfallen. Die Amide sollen ferner einen möglichst niedrigen Metallgehalt und eine geringe Eigenfärbung aufweisen. Zudem soll das Verfahren eine sichere und reproduzierbare Reaktionsführung gewährleisten.

Überraschenderweise wurde gefunden, dass sich Amide von Hydroxycarbonsäuren durch direkte Umsetzung von Hydroxycarbonsäuren mit Aminen in einem kontinuierlichen Verfahren durch nur kurzzeitiges Erhitzen mittels Bestrahlung mit Mikrowellen in einem Reaktionsrohr, dessen Längsachse sich in der Ausbreitungsrichtung der Mikrowellen eines Monomode-Mikrowellenapplikators befindet, in technisch relevanten Mengen herstellen lassen. Dabei wird die in den Mikrowellenapplikator eingestrahlte Mikrowellenenergie praktisch quantitativ vom Reaktionsgut absorbiert. Das erfindungsgemäße Verfahren besitzt zudem eine hohe Sicherheit bei der Durchführung und bietet eine hohe Reproduzierbarkeit der eingestellten Reaktionsbedingungen. Die nach dem erfindungsgemäßen Verfahren hergestellten Amide zeigen eine im Vergleich zu nach konventionellen Herstellverfahren ohne zusätzliche Verfahrensschritte nicht zugängliche hohe Reinheit und niedrige Eigenfärbung.

Gegenstand der Erfindung ist ein kontinuierliches Verfahren zur Herstellung von Hydroxycarbonsäureamiden, indem mindestens eine Hydroxycarbonsäure der Formel I

HO-R³-COOH (I)

worin R³ für einen gegebenenfalls substituierten aliphatischen Kohlenwasserstoffrest mit 1 bis 100 Kohlenstoffatomen steht,
mit mindestens einem Amin der Formel II

HNR¹R² (II)

worin R¹ und R² unabhängig voneinander für Wasserstoff oder einen Kohlenwasserstoffrest mit 1 bis 100 C-Atomen stehen,
zu einem Ammoniumsalz umgesetzt wird und dieses Ammoniumsalz nachfolgend unter Mikrowellenbestrahlung in einem Reaktionsrohr, dessen Längsachse sich in der Ausbreitungsrichtung der Mikrowellen eines Monomode-Mikrowellenapplikators befindet, zum Hydroxycarbonsäureamid umgesetzt wird.

Als Hydroxycarbonsäuren der Formel I sind allgemein Verbindungen geeignet, die mindestens eine Carboxylgruppe und mindestens eine Hydroxylgruppe an einem aliphatischen Kohlenwasserstoffrest R³ besitzen. So ist das erfindungsgemäße Verfahren ebenso zur Amidierung von Hydroxypolycarbonsäuren mit beispielsweise zwei, drei, vier oder mehr Carboxylgruppen geeignet. Bei der Umsetzung von Polycarbonsäuren mit Ammoniak oder primären Aminen gemäß dem erfindungsgemäßen Verfahren können auch Imide entstehen. Genauso ist das erfindungsgemäße Verfahren zur Amidierung von Polyhydroxycarbonsäuren mit beispielsweise zwei, drei, vier oder mehr Hydroxylgruppen geeignet, wobei die Hydroxycarbonsäuren jedoch nur eine Hydroxylgruppe pro C-Atom des aliphatischen Kohlenwasserstoffrestes R³ tragen dürfen. Besonders bevorzugt sind dabei Hydroxycarbonsäuren, die einen aliphatischen Kohlenwasserstoffrest R³ mit 1 bis 30 C-Atomen und insbesondere mit 2 bis 24 C-Atomen wie beispielsweise mit 3 bis 20 C-Atomen tragen. Der Kohlenwasserstoffrest R³ kann auch Heteroatome wie beispielsweise Sauerstoff, Stickstoff, Phosphor und/oder Schwefel enthalten, bevorzugt jedoch nicht mehr als ein Heteroatom pro 3 C-Atome. Die Hydroxycarbonsäuren der Formel I können natürlichen oder synthetischen Ursprungs sein.

Die aliphatischen Kohlenwasserstoffreste R³ können linear, verzweigt oder zyklisch sein. Die Carboxylgruppe kann an einem primären, sekundären oder tertiären C-Atom gebunden sein. Bevorzugt ist sie an einem primären C-Atom gebunden. Die Hydroxylgruppe kann an einem primären, sekundären oder tertiären C-Atom gebunden sein. Besonders vorteilhaft ist das Verfahren für die Amidierung von Hydroxycarbonsäuren, die eine Hydroxylgruppe an einem sekundären C-Atom gebunden enthalten und speziell für die Amidierung von solchen Hydroxycarbonsäuren, bei denen sich die Hydroxylgruppe in α-Position zur Carboxylgruppe befindet. Carboxyl- und Hydroxylgruppe können an gleiche oder verschiedene C-Atome von R³ gebunden sein. Die Kohlenwasserstoffreste R³ können gesättigt oder ungesättigt sein. Ungesättigte Kohlenwasserstoffreste R³ enthalten eine oder mehrere und bevorzugt eine, zwei oder drei C=C-Doppelbindungen. Bevorzugt befindet sich keine Doppelbindung in α,β-Position zur Carboxylgruppe. Bevorzugte zyklische aliphatische Kohlenwasserstoffreste besitzen mindestens einen Ring mit vier, fünf, sechs, sieben, acht oder mehr Ringatomen. Der Kohlenwasserstoffrest R³ kann einen oder mehrere wie beispielsweise zwei, drei, vier oder mehr weitere Substituenten tragen. Solche Substituenten können beispielsweise Halogenatome, halogenierte Alkylreste, C₁-C₅-Alkoxy-, wie beispielsweise Methoxy-, Poly(C₁-C₅-Alkoxy)-, Poly(C₁-C₅-Alkoxy)alkyl-, Carboxyl-, Hydroxyl-, Ester-, Amid-, Cyano-, Nitril-, Nitro- und/oder Arylgruppen mit 5 bis 20 Kohlenstoffatomen, wie beispielsweise Phenylgruppen, sein, mit der Maßgabe, dass die Substituenten unter den Reaktionsbedingungen stabil sind und keine Nebenreaktionen wie beispielsweise Eliminierungsreaktionen eingehen. Die C₅-C₂₀-Arylgruppen können ihrerseits wiederum Substituenten tragen. Solche Substituenten können beispielsweise Halogenatome, halogenierte Alkylreste, C₁-C₂₀-Alkyl-, C₂-C₂₀-Alkenyl-, C₁-C₅-Alkoxy-, wie beispielsweise Methoxy-, Ester-, Amid-, Hydroxy-, Cyano-, Nitril-, und/oder Nitrogruppen sein. Der Alkylrest trägt jedoch höchstens so viele Substituenten wie er Valenzen hat.

Besonders vorteilhaft ist das erfindungsgemäße Verfahren für die Herstellung von Amiden und insbesondere für die Herstellung von tertiären Amiden niederer Hydroxymonocarbonsäuren mit aliphatischen C₁-C₄-Kohlenwasserstoffresten erwiesen. Ebenfalls besonders vorteilhaft ist das Verfahren für die Herstellung von Hydroxypolycarbonsäuren mit insgesamt 3 bis 6 C-Atomen (inklusive der Carboxyl-Kohlenstoffatome) und 1 oder 2 Hydroxylgruppen.

Geeignete aliphatische Hydroxycarbonsäuren sind beispielsweise Hydroxyessigsäure, 2-Hydroxypropionsäure, 3-Hydroxypropionsäure, 2-Hydroxybuttersäure, 3-Hydroxybuttersäure, 4-Hydroxybuttersäure, 2-Hydroxy-2-methylpropionsäure, 4-Hydroxypentansäure, 5-Hydroxypentansäure, 2,2-Dimethyl-3-hydroxypropionsäure, 5-Hydroxyhexansäure, 2-Hydroxyoctansäure, 2-Hydroxytetradecansäure, 15-Hydroxypentadecansäure, 16-Hydroxyhexadecansäure, 12-Hydroxystearinsäure (Rizinolsäure) sowie α-Hydroxyphenylessigsäure (Mandelsäure), 4-Hydroxymandelsäure, 2-Hydroxy-2-phenylpropionsäure, und 3-Hydroxy-3-phenylpropionsäure. Auch Hydroxypolycarbonsäuren wie beispielsweise Hydroxybernsteinsäure, Zitronensäure und Isozitronensäure, Polyhydroxycarbonsäuren wie beispielsweise Gluconsäure und Mevalonsäure (3,5-Dihydroxy-3-methylpentansäure) sowie Polyhydroxypolycarbonsäuren wie beispielsweise Weinsäure lassen sich mittels des erfindungsgemäßen Verfahrens in die entsprechenden Diamide überführen. Erfindungsgemäß besonders bevorzugte Hydroxycarbonsäuren sind Hydroxyessigsäure, 2-Hydroxypropionsäure, Mandelsäure und Rizinolsäure.

Auch Mischungen verschiedener Hydroxycarbonsäuren sind für den Einsatz im erfindungsgemäßen Verfahren geeignet.

Das erfindungsgemäße Verfahren eignet sich bevorzugt zur Herstellung sekundärer Hydroxycarbonsäureamide, das heißt zur Umsetzung von Hydroxycarbonsäuren mit Aminen, bei denen R¹ für einen Kohlenwasserstoffrest mit 1 bis 100 Kohlenstoffatomen und R² für Wasserstoff steht.

Das erfindungsgemäße Verfahren eignet sich besonders bevorzugt zur Herstellung tertiärer Hydroxycarbonsäureamide, das heißt zur Umsetzung von Hydroxycarbonsäuren mit Aminen, bei denen beide Reste R¹ und R² unabhängig voneinander für einen Kohlenwasserstoffrest mit 1 bis 100 Kohlenstoffatomen stehen. Die Reste R¹ und R² können dabei gleich oder verschieden sein. In einer besonders bevorzugten Ausführungsform sind R¹ und R² gleich.

In einer ersten bevorzugten Ausführungsform stehen R¹ und/oder R² unabhängig voneinander für einen aliphatischen Rest. Dieser hat bevorzugt 1 bis 24, besonders bevorzugt 2 bis 18 und speziell 3 bis 6 C-Atome. Der aliphatische Rest kann linear, verzweigt oder zyklisch sein. Er kann weiterhin gesättigt oder ungesättigt sein. Der Kohlenwasserstoffrest kann Substituenten wie beispielsweise C₁-C₅-Alkoxy-, Cyano-, Nitril-, Nitro- und/oder C₅-C₂₀-Arylgruppen wie beispielsweise Phenylreste tragen. Die C₅-C₂₀-Arylreste können ihrerseits gegebenenfalls mit Halogenatomen, halogenierten Alkylresten, C₁-C₂₀-Alkyl-, C₂-C₂₀-Alkenyl-, C₁-C₅-Alkoxy- wie beispielsweise Methoxy-, Ester-, Amid-, Cyano-, Nitril-, und/oder Nitrogruppen substituiert sein. Besonders bevorzugte aliphatische Reste sind Methyl, Ethyl, n-Propyl, iso-Propyl, n-Butyl, iso-Butyl und tert.-Butyl, n-Hexyl, Cyclohexyl, n-Octyl, n-Decyl, n-Dodecyl, Tridecyl, Isotridecyl, Tetradecyl, Hexadecyl, Octadecyl und Methylphenyl. In einer besonders bevorzugten Ausführungsform stehen R¹ und/oder R² unabhängig voneinander für Wasserstoff, einen C₁-C₆-Alkyl-, C₂-C₆-Alkenyl- oder C₃-C₆-Cycloalkylrest und speziell für einen Alkylrest mit 1, 2, oder 3 C-Atomen. Diese Reste können bis zu drei Substituenten tragen.

In einer weiteren bevorzugten Ausführungsform bilden R¹ und R² zusammen mit dem Stickstoffatom, an das sie gebunden sind, einen Ring. Dieser Ring hat bevorzugt 4 oder mehr, wie beispielsweise 4, 5, 6 oder mehr Ringglieder. Bevorzugte weitere Ringglieder sind dabei Kohlenstoff-, Stickstoff-, Sauerstoff- und Schwefelatome. Die Ringe können ihrerseits wiederum Substituenten wie beispielsweise Alkylreste tragen. Geeignete Ringstrukturen sind beispielsweise Morpholinyl-, Pyrrolidinyl, Piperidinyl-, Imidazolyl- und Azepanylreste.

In einer weiteren bevorzugten Ausführungsform stehen R¹ und/oder R² unabhängig voneinander für eine gegebenenfalls substituierte C₆-C₁₂-Arylgruppe oder eine gegebenenfalls substituierte heteroaromatische Gruppe mit 5 bis 12 Ringgliedern.

In einer weiteren bevorzugten Ausführungsform stehen R¹ und/oder R² unabhängig voneinander für einen mit Heteroatomen unterbrochenen Alkylrest. Besonders bevorzugte Heteroatome sind Sauerstoff und Stickstoff.

So stehen R¹ und/oder R² unabhängig voneinander bevorzugt für Reste der Formel III

-(R⁴-O)ₙ-R⁵ (III)

worin
- R⁴: für eine Alkylengruppe mit 2 bis 6 C-Atomen und bevorzugt mit 2 bis 4 C-Atomen wie beispielsweise Ethylen, Propylen, Butylen oder Mischungen daraus,
- R⁵: für einen Kohlenwasserstoffrest mit 1 bis 24 C-Atomen oder für NR¹⁰R¹¹, und
- n: für eine Zahl zwischen 2 und 50, bevorzugt zwischen 3 und 25 und insbesondere zwischen 4 und 10 und
- R¹⁰, R¹¹: unabhängig voneinander für Wasserstoff, einen aliphatischen Rest mit 1 bis 24 C-Atomen und bevorzugt 2 bis 18 C-Atomen, eine Arylgruppe- oder Heteroarylgruppe mit 5 bis 12 Ringgliedern, eine Poly(oxyalkylen)gruppe mit 1 bis 50 Poly(oxyalkylen)einheiten, wobei sich die Polyoxyalkyleneinheiten von Alkylenoxideinheiten mit 2 bis 6 C-Atomen ableiten, oder R¹⁰ und R¹¹gemeinsam mit dem Stickstoffatom, an das sie gebunden sind, einen Ring mit 4, 5, 6 oder mehr Ringgliedern bilden, stehen.

Weiterhin bevorzugt stehen R¹ und/oder R² unabhängig voneinander für Reste der Formel IV

-[R⁶-N(R⁷)]ₘ-(R⁷) (IV)

worin
- R⁶: für eine Alkylengruppe mit 2 bis 6 C-Atomen und bevorzugt mit 2 bis 4 C-Atomen wie beispielsweise Ethylen, Propylen oder Mischungen daraus,
- jedes R⁷: unabhängig voneinander für Wasserstoff, einen Alkyl- oder Hydroxyalkylrest mit bis zu 24 C-Atomen wie beispielsweise 2 bis 20 C-Atomen, einen Polyoxyalkylenrest -(R⁴-O)ₚ-R⁵, oder einen Polyiminoalkylenrest -[R⁶-N(R⁷)]_{q}-(R⁷) stehen, wobei R⁴, R⁵, R⁶ und R⁷ die oben gegebenen Bedeutungen haben und q und p unabhängig voneinander für 1 bis 50, und
- m: für eine Zahl von 1 bis 20 und bevorzugt 2 bis 10 wie beispielsweise drei, vier, fünf oder sechs stehen. Die Reste der Formel IV enthalten vorzugsweise 1 bis 50, insbesondere 2 bis 20 Stickstoffatome.

Je nach stöchiometrischem Verhältnis zwischen Hydrocarbonsäure (I) und Polyamin (IV) werden ein oder mehrere Aminogruppen, die jeweils mindestens ein Wasserstoffatom tragen, in das Carbonsäureamid überführt. Bei der Umsetzung von Polycarbonsäuren mit Polyaminen der Formel IV können insbesondere die primären Aminogruppen auch in Imide überführt werden.

Zur erfindungsgemäßen Herstellung von primären Amiden werden anstelle von Ammoniak bevorzugt stickstoffhaltige Verbindungen, die beim Erhitzen Ammoniakgas abspalten, eingesetzt. Beispiele für derartige stickstoffhaltige Verbindungen sind Harnstoff und Formamid.

Beispiele für geeignete Amine sind Ammoniak, Methylamin, Ethylamin, Propylamin, Butylamin, Hexylamin, Cyclohexylamin, Octylamin, Cyclooctylamin, Decylamin, , 2-Cyclohexylethylamin Dodecylamin, Tetradecylamin, Hexadecylamin, Octadecylamin, Dimethylamin, Diethylamin, Ethylmethylamin, Di-n-propylamin, Di-iso-propylamin, Dicyclohexylamin, Didecylamin, Didodecylamin, Ditetradecylamin, Dihexadecylamin, Dioctadedcylamin, Benzylamin, Phenylethylamin, Ethylendiamin, Diethylentriamin, Triethylentetramin, Tetraethylenpentamin, Piperidin und Pyrrolidin sowie deren Mischungen. Besonders bevorzugt sind davon Dimethylamin, Diethylamin, Di-n-propylamin, Di-iso-propylamin und Ethylmethylamin.

Das Verfahren ist insbesondere geeignet zur Herstellung von N-Methylglycolsäureamid, N-Ethylmandelsäureamid N,N-Dimethylglycolsäureamid, N,N-Dimethylmilchsäureamid, und N, N-Dimethylricinolsäureamid.

Im erfindungsgemäßen Verfahren wird die Hydroxycarbonsäure bevorzugt mit mindestens equimolaren Mengen Amin und besonders bevorzugt mit einem Überschuss an Amin umgesetzt, das heißt die molaren Verhältnisse von Aminogruppen zu Carboxylgruppen betragen mindestens 1:1 und liegen bevorzugt zwischen 100:1 und 1,001:1, besonders bevorzugt zwischen 10:1 und 1,01:1 wie beispielsweise zwischen 5:1 und 1,1:1. Dabei werden die Carboxylgruppen praktisch quantitativ zum Amid umgesetzt ohne dass es zur Aminolyse von Hydroxylgruppen kommt. Besonders vorteilhaft ist dieses Verfahren, wenn das eingesetzte Amin leicht flüchtig ist. Leicht flüchtig heißt hier, dass das Amin einen Siedepunkt bei Normaldruck von vorzugsweise unterhalb 200 °C wie beispielsweise unterhalb 160 °C besitzt und sich somit destillativ vom Amid abtrennen lässt. In einer speziellen Ausführungsform werden Carbonsäure und Amin equimolar eingesetzt.

Die erfindungsgemäße Herstellung der Amide erfolgt durch Umsetzung von Carbonsäure und Amin zum Ammoniumsalz und nachfolgender Bestrahlung des Salzes mit Mikrowellen in einem Reaktionsrohr, dessen Längsachse sich in der Ausbreitungsrichtung der Mikrowellen in einem Monomode-Mikrowellenapplikator befindet.

Bevorzugt erfolgt die Bestrahlung des Salzes mit Mikrowellen in einem weitgehend mikrowellentransparenten Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Weiterhin bevorzugt werden die im Hohlleiter nicht absorbierten Mikrowellen an seinem Ende reflektiert. Durch Ausformung des Mikrowellenapplikators als Resonator vom Reflexionstyp werden eine lokale Erhöhung der elektrischen Feldstärke durch Interferenz bei gleicher vom Generator zugeführter Leistung und eine erhöhte Energieausnutzung erzielt.

Der Hohlraumresonator wird bevorzugt im E₀₁ₙ-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert null ab. Diese Feldkonfiguration liegt rotationssymmetrisch um die zentrale Symmetrieachse vor. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 4 bis 50 und speziell von 3 bis 20 wie beispielsweise 3, 4, 5, 6, 7 oder 8.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Ammoniumsalzes mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators, wozu der Hohlraumresonator bevorzugt je eine zentrische Öffnung an zwei gegenüber liegenden Stirnwänden zum Hindurchführen des Reaktionsrohres aufweist.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Salzes mit Mikrowellen in einem mikrowellentransparenten Reaktionsrohr, das sich axialsymmetrisch in einem E₀₁ₙ-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Salzes mit Mikrowellen in einem mikrowellentransparenten Reaktionsrohr, das durch einen E₀₁ₙ-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Salzes mit Mikrowellen in einem mikrowellentransparenten Reaktionsrohr, das sich axialsymmetrisch in einem kreiszylindrischen E₀₁ₙ-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden.

Mikrowellengeneratoren, wie beispielsweise das Magnetron, das Klystron und das Gyrotron sind dem Fachmann bekannt.

Die zur Durchführung des erfindungsgemäßen Verfahrens eingesetzten Reaktionsrohre sind bevorzugt aus weitgehend mikrowellentransparentem, hoch schmelzendem Material gefertigt. Besonders bevorzugt werden nichtmetallische Reaktionsrohre eingesetzt. Unter weitgehend mikrowellentransparent werden hier Werkstoffe verstanden, die möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan δ = ε"/ε' herangezogen. Der dielektrische Verlustfaktor tan δ ist definiert als das Verhältnis aus dielektrischem Verlust ε" und Dielektrizitätskonstante ε'. Beispiele für tan δ-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan δ-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Zirkonoxid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Für das erfindungsgemäße Verfahren besonders geeignete Reaktionsrohre haben einen Innendurchmesser von einem Millimeter bis ca. 50 cm, speziell zwischen 2 mm und 35 cm wie beispielsweise zwischen 5 mm und 15 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Reaktionsrohres verstanden, auf der die Mikrowellenbestrahlung erfolgt. In das Reaktionsrohr können Stromstörer und/oder andere Mischelemente eingebaut sein.

Für das erfindungsgemäße Verfahren besonders geeignete E₀₁-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der E₀₁-Hohlraumresonator einen runden Querschnitt, was auch als E₀₁-Rundhohlleiter bezeichnet wird. Besonders bevorzugt hat er eine zylindrische Form und speziell eine kreiszylindrische Form.

Das Reaktionsrohr ist üblicherweise am Einlass mit einer Dosierpumpe sowie einem Manometer und am Auslass mit einer Druckhaltevorrichtung und einem Wärmetauscher versehen. Damit sind Reaktionen in einem sehr weiten Druck- und Temperaturbereich möglich.

Die Umsetzung von Amin und Carbonsäure zum Ammoniumsalz kann kontinuierlich, diskontinuierlich oder auch in semi-Batch-Prozessen durchgeführt werden. So kann die Herstellung des Ammoniumsalzes in einem vorgelagerten (semi)-Batch Prozess durchgeführt werden wie beispielsweise in einem Rührbehälter. Das Ammoniumsalz wird bevorzugt in-situ erzeugt und nicht isoliert. In einer bevorzugten Ausführungsform werden die Edukte Amin und Carbonsäure, beide unabhängig voneinander gegebenenfalls mit Lösemittel verdünnt, erst kurz vor dem Eintritt in das Reaktionsrohr vermischt. So hat es sich besonders bewährt, die Umsetzung von Amin und Carbonsäure zum Ammoniumsalz in einer Mischstrecke vorzunehmen, aus der das Ammoniumsalz, gegebenenfalls nach Zwischenkühlung, in das Reaktionsrohr gefördert wird. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein Katalysator kann, sofern eingesetzt, einem der Edukte oder auch der Eduktmischung vor dem Eintritt in das Reaktionsrohr zugesetzt werden. Auch feste, pulverförmige und heterogene Systeme können nach dem erfindungsgemäßen Verfahren umgesetzt werden, wobei lediglich entsprechende technische Vorrichtungen zum Fördern des Reaktionsgutes erforderlich sind.

Das Ammoniumsalz kann in das Reaktionsrohr entweder an dem durch das Innenleiterrohr geführte Ende eingespeist werden als auch an dem entgegen gesetzten Ende.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators, der eingestrahlten Mikrowellenleistung sowie der dabei erreichten Temperatur werden die Reaktionsbedingungen so eingestellt, dass die maximale Reaktionstemperatur schnellstmöglich erreicht wird und die Verweilzeit bei Maximaltemperatur so kurz bleibt, dass so wenig Neben- oder Folgereaktionen wie möglich auftreten. Das Reaktionsgut kann zur Vervollständigung der Reaktion, gegebenenfalls nach Zwischenkühlung, mehrfach das Reaktionsrohr durchlaufen. Vielfach hat sich bewährt, wenn das Reaktionsprodukt unmittelbar nach Verlassen des Reaktionsrohres z. B. durch Mantelkühlung oder Entspannung abgekühlt wird. Bei langsamer verlaufenden Reaktionen hat es sich oftmals bewährt, das Reaktionsprodukt nach Verlassen des Reaktionsrohres noch eine gewisse Zeit bei Reaktionstemperatur zu halten.

Die Vorteile des erfindungsgemäßen Verfahrens liegen in einer sehr gleichmäßigen Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines E₀₁-Hohlraumresonators beispielsweise mit koaxialem Übergang befindet. Dabei erlaubt das erfindungsgemäße Reaktordesign die Durchführung von Reaktionen auch bei sehr hohen Drücken und/oder Temperaturen. Durch Temperatur- und/oder Druckerhöhung wird eine deutliche Steigerung von Umsetzungsgrad und Ausbeute auch gegenüber bekannten Mikrowellenreaktoren beobachtet, ohne dass es dabei zu unerwünschten Nebenreaktionen und/oder Verfärbungen kommt. Überraschenderweise wird dabei wird ein sehr hoher Wirkungsgrad bei der Ausnutzung der in den Hohlraumresonator eingestrahlten Mikrowellenenergie erzielt, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Das erfindungsgemäße Verfahren erlaubt zudem eine kontrollierte, sichere und reproduzierbare Reaktionsführung. Da das Reaktionsgut im Reaktionsrohr parallel zur Ausbreitungsrichtung der Mikrowellen bewegt wird, werden bekannte Überhitzungsphänomene durch unkontrollierbare Feldverteilungen, die zu lokalen Überhitzungen durch wechselnde Intensitäten des Feldes beispielsweise in Wellenbergen und Knotenpunkten führen, durch die Fließbewegung des Reaktionsgutes ausgeglichen. Die genannten Vorteile erlauben es auch, mit hohen Mikrowellenleistungen von beispielsweise mehr als 10 kW oder mehr als 100 kW zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei war es besonders überraschend, dass trotz der im kontinuierlich durchflossenen Strömungsrohr nur sehr kurzen Verweildauer des Ammoniumsalzes im Mikrowellenfeld eine sehr weitgehende Amidierung mit Umsätzen im allgemeinen von über 80 %, oftmals auch über 90 % wie beispielsweise über 95 % bezogen auf die im Unterschuss eingesetzte Komponente stattfindet, ohne Bildung nennenswerter Mengen an Nebenprodukten. Trotz Anwesenheit von OH Gruppen mit alkoholischem Charakter in den erfindungsgemäßen Säurestrukturen, werden diese weder von der Säurekomponente selbst (Esterbildung) noch von der Aminfunktion (Aminolyse) angegriffen. So konnten weder Ester noch Polyester als Nebenprodukte gefunden werden. Bei einer entsprechenden Umsetzung dieser Ammoniumsalze in einem Strömungsrohr gleicher Dimensionierung unter thermischer Mantelheizung werden zur Erzielung geeigneter Reaktionstemperaturen extrem hohe Wandtemperaturen benötigt, die zur Bildung gefärbter Spezies führten, aber bei gleichem Zeitintervall nur geringfügige Amidbildung bewirken. Des Weiteren besitzen die nach dem erfindungsgemäßen Verfahren hergestellten Produkte sehr niedrige Metallgehalte ohne dass es einer weiteren Aufarbeitung der Rohprodukte bedarf. So liegen die Metallgehalte der nach dem erfindungsgemäßen Verfahren hergestellten Produkte bezogen auf Eisen als Hauptelement üblicherweise unterhalb 25 ppm bevorzugt unterhalb 15 ppm, speziell unterhalb 10 ppm wie beispielsweise zwischen 0,01 und 5 ppm Eisen.

Bevorzugt wird der durch die Mikrowellenbestrahlung bedingte Temperaturanstieg beispielsweise durch Regelung der Mikrowellenintensität, der Durchflussgeschwindigkeit und/oder durch Kühlung des Reaktionsrohres, beispielsweise durch einen Stickstoffstrom, auf maximal 500 °C begrenzt. Besonders bewährt hat sich die Durchführung der Umsetzung bei Temperaturen zwischen 150 und maximal 400 °C und insbesondere zwischen 180 und maximal 350 °C und speziell zwischen 200 und 320 °C wie beispielsweise bei Temperaturen zwischen 220 und 300 °C.

Die Dauer der Mikrowellenbestrahlung hängt von verschiedenen Faktoren wie beispielsweise der Geometrie des Reaktionsrohres, der eingestrahlten Mikrowellenenergie, der speziellen Reaktion und dem gewünschten Umsetzungsgrad ab. Üblicherweise wird die Mikrowellenbestrahlung über einen Zeitraum von weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten vorgenommen. Die Intensität (Leistung) der Mikrowellenstrahlung wird dabei so eingestellt, dass das Reaktionsgut beim Verlassen des Hohlraumresonators die gewünschte Maximaltemperatur hat. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt.

Bevorzugt wird die Umsetzung bei Drücken zwischen 0,01 und 500 bar und besonders bevorzugt zwischen 1 bar (Atmosphärendruck) und 150 bar und speziell zwischen 1,5 bar und 100 bar wie beispielsweise zwischen 3 bar und 50 bar durchgeführt. Besonders bewährt hat sich das Arbeiten unter erhöhtem Druck, wobei oberhalb des Siedepunkts (bei Normaldruck) der Edukte bzw. Produkte, des gegebenenfalls anwesenden Lösemittels und/oder oberhalb des während der Reaktion gebildeten Reaktionswassers gearbeitet wird. Besonders bevorzugt wird der Druck so hoch eingestellt, dass das Reaktionsgemisch während der Mikrowellenbestrahlung im flüssigen Zustand verbleibt und nicht siedet.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

In einer bevorzugten Ausführungsform wird zur Beschleunigung bzw. zur Vervollständigung der Reaktion in Gegenwart von dehydratisierenden Katalysatoren gearbeitet. Vorzugsweise arbeitet man dabei in Gegenwart eines sauren anorganischen, metallorganischen oder organischen Katalysators oder Gemischen aus mehreren dieser Katalysatoren.

Als saure anorganische Katalysatoren im Sinne der vorliegenden Erfindung sind beispielsweise Schwefelsäure, Phosphorsäure, Phosphonsäure, hypophosphorige Säure, Aluminiumsulfathydrat, Alaun, saures Kieselgel und saures Aluminiumhydroxid zu nennen. Weiterhin sind beispielsweise Aluminiumverbindungen der allgemeinen Formel Al(OR¹⁵)₃ und Titanate der allgemeinen Formel Ti(OR¹⁵)₄ als saure anorganische Katalysatoren einsetzbar, wobei die Reste R¹⁵ jeweils gleich oder verschieden sein können und unabhängig voneinander gewählt sind aus C₁-C₁₀-Alkylresten, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R¹⁵ in Al(OR¹⁵)₃ bzw. Ti(OR¹⁵)₄ jeweils gleich und gewählt aus Isopropyl, Butyl und 2-Ethylhexyl.

Bevorzugte saure metallorganische Katalysatoren sind beispielsweise gewählt aus Dialkylzinnoxiden (R¹⁵)₂SnO, wobei R¹⁵ wie oben stehend definiert ist. Ein besonders bevorzugter Vertreter für saure metallorganische Katalysatoren ist Di-n-butylzinnoxid, das als sogenanntes Oxo-Zinn oder als Fascat^{®}-Marken kommerziell erhältlich ist.

Bevorzugte saure organische Katalysatoren sind saure organische Verbindungen mit beispielsweise Phosphatgruppen, Sulfonsäuregruppen, Sulfatgruppen oder Phosphonsäuregruppen. Besonders bevorzugte Sulfonsäuren enthalten mindestens eine Sulfonsäuregruppe und mindestens einen gesättigten oder ungesättigten, linearen, verzweigten und/oder zyklischen Kohlenwasserstoffrest mit 1 bis 40 C-Atomen und bevorzugt mit 3 bis 24 C-Atomen. Insbesondere bevorzugt sind aromatische Sulfonsäuren, speziell alkylaromatische Mono-Sulfonsäuren mit einem oder mehreren C₁-C₂₈-Alkylresten und insbesondere solche mit C₃-C₂₂-Alkylresten. Geeignete Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure; Dodecylbenzolsulfonsäure, Didodecylbenzolsulfonsäure, Naphthalinsulfonsäure. Auch saure Ionenaustauscher können als saure organische Katalysatoren eingesetzt werden, beispielsweise Sulfonsäuregruppenhaltige Poly(styrol)-Harze, die mit etwa 2 mol-% Divinylbenzol vernetzt sind.

Besonders bevorzugt für die Durchführung des erfindungsgemäßen Verfahrens sind Borsäure, Phosphorsäure, Polyphosphorsäure und Polystyrolsulfonsäuren. Insbesondere bevorzugt sind Titanate der allgemeinen Formel Ti(OR¹⁵)₄ und speziell Titantetrabutylat und Titantetraisopropylat.

Wünscht man saure anorganische, metallorganische oder organische Katalysatoren einzusetzen, so setzt man erfindungsgemäß 0,01 bis 10 Gew.-%, bevorzugt 0,02 bis 2 Gew.-% Katalysator ein. In einer besonders bevorzugten Ausführungsform wird ohne Katalysator gearbeitet.

In einer weiteren bevorzugten Ausführungsform wird die Mikrowellenbestrahlung in Gegenwart von sauren, festen Katalysatoren durchgeführt. Dabei wird der feste Katalysator in dem gegebenenfalls mit Lösemittel versetzten Ammoniumsalz suspendiert oder vorteilhafterweise das gegebenenfalls mit Lösemittel versetzte Ammoniumsalz über einen Festbettkatalysator geleitet und Mikrowellenstrahlung ausgesetzt. Geeignete feste Katalysatoren sind beispielsweise Zeolithe, Kieselgel, Montmorillonit und (teil)vernetzte Polystyrolsulfonsäure, die gegebenenfalls mit katalytisch aktiven Metallsalzen imprägniert sein können. Geeignete saure Ionentauscher auf Basis von Polystyrolsulfonsäuren, die als Festphasenkatalysatoren eingesetzt werden können, sind beispielsweise von der Firma Rohm & Haas unter der Markenbezeichnung Amberlyst^{®} erhältlich.

Es hat sich bewährt, in Gegenwart von Lösemitteln zu arbeiten um beispielsweise die Viskosität des Reaktionsmediums abzusenken und/oder das Reaktionsgemisch, sofern es heterogen ist, zu fluidisieren. Dafür können prinzipiell alle Lösemittel eingesetzt werden, die unter den angewendeten Reaktionsbedingungen inert sind und nicht mit den Edukten bzw. den gebildeten Produkten reagieren. Ein wichtiger Faktor bei der Auswahl geeigneter Lösemittel ist deren Polarität, die einerseits die Löseeigenschaften und andererseits das Ausmaß der Wechselwirkung mit Mikrowellenstrahlung bestimmt. Ein besonders wichtiger Faktor bei der Auswahl geeigneter Lösemittel ist deren dielektrischer Verlust ε". Der dielektrische Verlust ε" beschreibt den Anteil an Mikrowellenstrahlung, der bei der Wechselwirkung einer Substanz mit Mikrowellenstrahlung in Wärme überführt wird. Letzt genannter Wert hat sich als besonders wichtiges Kriterium für die Eignung eines Lösemittels für die Durchführung des erfindungsgemäßen Verfahrens erwiesen. Besonders bewährt hat sich das Arbeiten in Lösemitteln, die eine möglichst geringe Mikrowellenabsorption zeigen und somit nur einen kleinen Beitrag zur Erwärmung des Reaktionssystems liefern. Für das erfindungsgemäße Verfahren bevorzugte Lösemittel besitzen einen bei Raumtemperatur und 2450 MHz gemessenen dielektrischen Verlust ε" von weniger als 10 und vorzugsweise weniger als 1 wie beispielsweise weniger als 0,5. Eine Übersicht über den dielektrischen Verlust verschiedener Lösemittel findet sich zum Beispiel in "Microwave Synthesis" von B. L. Hayes, CEM Publishing 2002. Für das erfindungsgemäße Verfahren geeignet sind insbesondere Lösemittel mit ε"-Werten unterhalb 10 wie N-Methylpyrrolidon, N,N-Dimethylformamid oder Aceton, und insbesondere Lösemittel mit ε"-Werten unterhalb 1. Beispiele für besonders bevorzugte Lösemittel mit ε"-Werten unterhalb 1 sind aromatische und/oder aliphatische Kohlenwasserstoffe wie beispielsweise Toluol, Xylol, Ethylbenzol, Tetralin, Hexan, Cyclohexan, Decan, Pentadecan, Dekalin sowie kommerzielle Kohlenwasserstoffgemische wie Benzinfraktionen, Kerosin, Solvent Naphtha, Shellsol^{®} AB, Solvesso^{®} 150, Solvesso^{®} 200, Exxsol^{®}, Isopar^{®} und Shellsol^{®}-Typen. Lösemittelgemische, die ε"-Werte bevorzugt unterhalb 10 und speziell unterhalb 1 aufweisen, sind für die Durchführung des erfindungsgemäßen Verfahrens gleichermaßen bevorzugt.

Prinzipiell ist das erfindungsgemäße Verfahren auch in Lösemitteln mit höheren ε"-Werten von beispielsweise 5 und höher wie insbesondere mit ε"-Werten von 10 und höher durchführbar. Die dabei beobachtete beschleunigte Aufheizung des Reaktionsgemischs erfordert jedoch besondere Maßnahmen zur Einhaltung der Maximaltemperatur.

Sofern in Gegenwart von Lösemitteln gearbeitet wird, liegt deren Anteil an der Reaktionsmischung bevorzugt zwischen 2 und 95 Gew.-%, speziell zwischen 5 und 90 Gew.-% und insbesondere zwischen 10 und 75 Gew.-% wie beispielsweise zwischen 30 und 60 Gew.-%. Besonders bevorzugt wird die Reaktion lösemittelfrei durchgeführt.

Als Mikrowellen werden elektromagnetische Strahlen mit einer Wellenlänge zwischen etwa 1 cm und 1m und Frequenzen zwischen etwa 300 MHz und 30 GHz bezeichnet. Dieser Frequenzbereich ist prinzipiell für das erfindungsgemäße Verfahren geeignet. Bevorzugt wird für das erfindungsgemäße Verfahren Mikrowellenstrahlung mit den für industrielle, wissenschaftliche und medizinische Anwendungen freigegebenen Frequenzen verwendet wie beispielsweise mit Frequenzen von 915 MHz, 2,45 GHz, 5,8 GHz oder 27,12 GHz.

Die für die Durchführung des erfindungsgemäßen Verfahrens in den Hohlraumresonator einzustrahlende Mikrowellenleistung ist insbesondere abhängig von der Geometrie des Reaktionsrohrs und damit des Reaktionsvolumens sowie der Dauer der erforderlichen Bestrahlung. Sie liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden.

In einer bevorzugten Ausführungsform wird die Reaktion in einem druckfesten inert-Rohr durchgeführt, wobei das sich bildende Reaktionswasser sowie gegebenenfalls Edukte und, sofern anwesend, Lösemittel zu einem Druckaufbau führen. Nach Beendigung der Reaktion kann der Überdruck durch Entspannung zur Verflüchtigung und Abtrennung von Reaktionswasser, überschüssigen Edukten sowie gegebenenfalls Lösemittel und/oder zur Abkühlung des Reaktionsprodukts verwendet werden. In einer weiteren Ausführungsform wird das gebildete Reaktionswasser nach dem Abkühlen und/oder Entspannen durch übliche Verfahren wie beispielsweise Phasentrennung, Destillation Strippen, Flashen und/oder Absorption abgetrennt.

Zur Vervollständigung der Umsetzung hat es sich in vielen Fällen bewährt, das erhaltene Rohprodukt nach Entfernen von Reaktionswasser sowie gegebenenfalls Austragen von Produkt und/oder Nebenprodukt erneut der Mikrowellenbestrahlung auszusetzen, wobei gegebenenfalls das Verhältnis der eingesetzten Reaktanden um verbrauchte oder unterschüssige Edukte zu ergänzen ist.

Üblicherweise fallen über den erfindungsgemäßen Weg hergestellte Amide in einer für die weitere Verwendung ausreichenden Reinheit an. Für spezielle Anforderungen können sie jedoch nach üblichen Reinigungsverfahren wie beispielsweise Destillation, Umkristallisation, Filtration bzw. chromatographische Verfahren weiter aufgereinigt werden.

Das erfindungsgemäße Verfahren erlaubt eine sehr schnelle, energiesparende und kostengünstige Herstellung von Amiden niederer Carbonsäuren in hohen Ausbeuten und mit hoher Reinheit in großtechnischen Mengen. Durch die sehr gleichmäßige Bestrahlung des Ammoniumsalzes im Zentrum des rotationssymmetrischen Mikrowellenfeldes erlaubt es eine sichere, kontrollierbare und reproduzierbare Reaktionsführung. Dabei wird durch einen sehr hohen Wirkungsgrad bei der Ausnutzung der eingestrahlten Mikrowellenenergie eine den bekannten Herstellverfahren deutlich überlegene Wirtschaftlichkeit erzielt. Bei diesem Verfahren fallen keine wesentlichen Mengen an Nebenprodukten an. Derartig schnelle und selektive Umsetzungen sind nach klassischen Methoden nicht zu erzielen und waren alleine durch Heizen auf hohe Temperaturen nicht zu erwarten. Die nach dem erfindungsgemäßen Verfahren hergestellten Produkte sind oftmals so rein, dass keine weiteren Auf- oder Nacharbeitungsschritte erforderlich sind.

### Beispiele

Die Umsetzungen der Ammoniumsalze unter Mikrowellenbestrahlung erfolgten in einem Keramikrohr (60 x 1 cm), das sich axialsymmetrisch in einem zylindrischen Hohlraumresonator (60 x 10 cm) befand. An einer der Stirnseiten des Hohlraumresonators verlief das Keramikrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt (E₀₁-Hohlraumapplikator; Monomode).

Die Mikrowellenleistung wurde über die Versuchsdauer jeweils in der Art eingestellt, dass die gewünschte Temperatur des Reaktionsguts am Ende der Bestrahlungszone konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen der Reaktionszone (etwa 15 cm Strecke in einer isolierten Edelstahlkapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurück gespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet

Mittels einer Hochdruckpumpe und eines geeigneten Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die aus Carbonsäure und Amin hergestellten Ammoniumsalze wurden mit einer konstanten Flussrate durch das Reaktionsrohr gepumpt und die Verweilzeit in der Bestrahlungszone durch Modifizierung der Strömungsgeschwindigkeit eingestellt.

Die Analytik der Produkte erfolgte mittels ¹H-NMR-Spektroskopie bei 500 MHz in CDCl₃. Die Bestimmung von Eisengehalten erfolgte mittels Atomabsorptionsspektroskopie.

### Beispiel 1: Herstellung von Milchsäure-N,N-dimethylamid

Unter Trockeneiskühlung wurden in eine Kühlfalle 1,35 kg (30 mol) Dimethylamin aus einer Vorratsflasche einkondensiert. In einem Dreihalskolben mit Gaseinleitungsrohr, Rührer, Innenthermometer und Druckausgleich wurden 2,7 kg (30 mol) Milchsäure vorgelegt und auf 60 °C erwärmt. Durch langsames Auftauen der Kühlfalle wurde gasförmiges Dimethylamin durch das Gaseinleitungsrohr in den Dreihalskolben geleitet. In einer stark exothermen Reaktion bildete sich das Milchsäure-N,N-dimethylammonium Salz.

Das so erhaltene Ammoniumsalz wurde bei einem Arbeitsdruck von etwa 25 bar kontinuierlich mit 5,0 l/h durch das Reaktionsrohr gepumpt und einer Mikrowellenleistung von 1,9 kW ausgesetzt, von denen 94 % vom Reaktionsgut absorbiert wurden. Die Verweilzeit des Reaktionsgemischs in der Bestrahlungszone betrug ca. 34 Sekunden. Am Ende des Reaktionsrohres hatte das Reaktionsgemisch eine Temperatur von 235 °C.

Es wurde ein Umsatz von 94 % d. Th. erreicht. Das Reaktionsprodukt war leicht gelb gefärbt und enthielt < 2 ppm Eisen. Die bei der thermischen Reaktion zwangsanfallenden Nebenprodukte (siehe Vergleichsbeispiel 3) konnten nicht nachgewiesen werden (GC-Analyse, Nachweisgrenze 0,1 Gew.-%).

Nach destillativer Abtrennung des Reaktionswassers am Dünnschichtverdampfer wurde das Produkt bei einer Siedetemperatur von 105-108 °C (8 mbar) mit einer Reinheit von 99,5 % in 88 %iger Ausbeute isoliert. Im Sumpf verblieben die unreagierten Reste des Milchsäure-N,N-dimethylammonium Salzes, die bei erneuter Mikrowellenbestrahlung praktisch quantitativ in das Amid überführt werden konnten.

### Beispiel 2: Herstellung von Milchsäure-N,N-diethylamid

Unter Kühlen und Rühren wurde 2,7 kg (30 mol) Milchsäure langsam mit einem 1,05 molaren Überschuss an Diethylamin versetzt (2,3 kg, 31,5 mol) und gemischt (Gaseinleitung siehe Beispiel 1). Nach Abklingen der Wärmetönung wurde das so erhaltene Ammoniumsalz auf 80 °C temperiert und anschließend bei einem Arbeitsdruck von etwa 25 bar kontinuierlich mit 4,8 l/h durch das Reaktionsrohr gepumpt und einer Mikrowellenleistung von 2,4 kW ausgesetzt, von denen 95 % vom Reaktionsgut absorbiert wurden. Die Verweilzeit des Reaktionsgemischs in der Bestrahlungszone betrug ca. 35 Sekunden. Am Ende des Reaktionsrohres hatte das Reaktionsgemisch eine Temperatur von 258 °C.

Es wurde ein Umsatz von 97 % d. Th. erzielt. Das Reaktionsprodukt war dunkel gelb gefärbt und enthielt < 2 ppm Eisen. Das Produkt wurde am Dünnschichtverdampfer von Wasser und überschüssigem Amin befreit und konnte anschließend ohne weitere Reinigung eingesetzt werden.

### Beispiel 3: Herstellung von Milchsäure-N,N-dimethylamid durch thermische Kondensation (Vergleichsbeispiel)

In einem 500 ml Glaskolben mit Rührer und absteigendem Intensivkühler wurden 180 g (2,0 mol) Milchsäure vorgelegt und auf 60 °C erwärmt. Anschließend wurden 90,16 g (2,0 mol) Dimethylamin gasförmig in den Kolben eingeleitet. Durch Temperaturerhöhung auf 180 °C wurde die Reaktion in Gang gebracht. Das sich während der Reaktion bildende Kondensatwasser und unreagiertes Dimethylamin wurden kontinuierlich abdestilliert Nach 20 Stunden wurde die Reaktion beendet, nachdem sich kein weiteres Reaktionswasser mehr bildete.

Bezogen auf die eingesetzte Säure wurde ein Umsatz 90 % gefunden. Es kam während der Reaktion zu einer deutlichen Schwarzfärbung des Reaktionsgutes. Daneben bildeten sich unlösliche Anteile, die auf eine partielle Zersetzung des Reaktionsgutes hinwiesen. Als Nebenkomponenten wurden einerseits oligomere Milchsäurepolymere (5 %) und andererseits Aminolyseprodukte des Milchsäuredimethylamids (2-Dimethylamino-propionsäuredimethylamid) bzw. der Milchsäure (4 %) nachgewiesen. Weiterhin wurden im Abgas nicht weiter quantifizierte Mengen des Eliminierungsprodukts Acrylsäure-N,N-dimethylamid gefunden.

### Beispiel 4: Herstellung von Milchsäure-N,N-dimethylamid im diskontinuierlichen Single-Mode Labormikrowellenreaktor (Vergleich)

Es wurde eine Schmelze des Milchsäure-N,N-dimethylammonium Salzes nach der im vorangegangenen Beispiel beschriebenen Methode hergestellt.

Von dieser Schmelze wurden 2 ml in eine druckfeste Viole druckfest eingeschlossen und in die Mikrowellen-Kaverne eines "Biotage Initiator™" Labormikrowellengerätes eingebracht. Das Reaktionsgut wurde anschließend durch Anlegen einer Mikrowellenleistung von 250 Watt innerhalb von einer Minute auf 240 °C erhitzt, dabei bildete sich ein Druck von etwa 20 bar aus. Nach Ende der Aufheizzeit wurde weitere 4 Minuten mit geregelter Leistung auf die Probe eingestrahlt. Dabei wurde die Leistung in der Art angepasst, dass die Temperatur des Reaktionsgutes bei 240 °C konstant blieb.

Bezogen auf die eingesetzte Säure wurde im Rohprodukt ein Umsatz von 45 % gefunden. Das Produkt hatte eine dunkel-braune bis schwarze Farbe. Auch hier kam es zur Bildung von schwarzen Schwebeteilchen, die durch einen Filter abgetrennt werden konnten. Dieser Feststoff konnte in keinem gängigen Lösungsmittel gelöst werden, was als Indiz dafür genommen werden kann, dass es sich hier um Pyrolyseprodukte des Reaktionsgutes handelt. Polymere Strukturen, wie im vorangegangenen Vergleichsbeispiel beschrieben, wurden nicht beobachtet.

### Beispiel 5: Herstellung von 3-Hydroxybuttersäuredimethylamid

2,6 kg (25 mol) 3-Hydroxybutansäure wurden in einem 10 Liter Rührautoklaven (Büchi) in 2 Litern Toluol gelöst. Anschließend wurde die Säure unter Kühlung mittels Einleiten einer equimolaren Menge gasförmigen Dimethylamins in das Ammoniumsalz überführt (Vorgehen entsprechend Beispiel 1). Nach Abklingen der Wärmetönung wurde das so erhaltene Ammoniumsalz bei einem Arbeitsdruck von etwa 25 bar kontinuierlich mit 3,0 l/h durch das Reaktionsrohr gepumpt und einer Mikrowellenleistung von durchschnittlich 1,85 kW ausgesetzt, von denen 88 % vom Reaktionsgut absorbiert wurden. Die Verweilzeit des Reaktionsgemischs in der Bestrahlungszone betrug ca.57 Sekunden. Am Ende des Reaktionsrohres hatte das Reaktionsgemisch eine Temperatur von 244 °C.

Es wurde ein Umsatz von 92 % d. Th. erreicht. Das Reaktionsprodukt war braun gefärbt und enthielt keinerlei Anzeichen von Polymerbildung. Aminolyseprodukte wie 3-N,N-Dimethylaminobuttersäure oder 3-N,N-Dimethylaminobuttersäuredimethylamid konnten in der Rohproduktmischung nicht nachgewiesen werden. Der Gehalt an Eisen lag < 2 ppm Eisen. Das 3-Hydroxybuttersäuredimethylamid wurde durch Abdampfen von Toluols und des entstandenen Reaktionswassers isoliert.

### Beispiel 6: Herstellung von Ricinolsäuredimethylamid

2,98 kg (10 mol) 12-Hydroxy-9-octadecensäure (Ricinolsäure) wurden in einem 10 Liter Rührautoklaven (Büchi) in 2 Litern Toluol gelöst. Anschließend wurde die Säure unter Kühlung mittels Einleiten einer equimolaren Menge gasförmigen Dimethylamins (0,45 kg) in das Ammoniumsalz überführt (Vorgehen entsprechend Beispiel 1). Nach Abklingen der Wärmetönung wurde das so erhaltene Ammoniumsalz bei einem Arbeitsdruck von etwa 25 bar kontinuierlich mit 3,5 l/h durch das Reaktionsrohr gepumpt und einer Mikrowellenleistung von durchschnittlich 2,1 kW ausgesetzt, von denen 89 % vom Reaktionsgut absorbiert wurden. Die Verweilzeit des Reaktionsgemischs in der Bestrahlungszone betrug ca. 49 Sekunden. Am Ende des Reaktionsrohres hatte das Reaktionsgemisch eine Temperatur von 240 °C.

Es wurde ein Umsatz von 90 % d. Th. erreicht. Das Reaktionsprodukt war gelblich braun gefärbt. Aminolyseprodukte konnten auch bei diesem Beispiel in der Rohproduktmischung nicht nachgewiesen werden. Der Gehalt an Eisen war < 2 ppm. Trotz hoher Reaktionstemperatur wurden im Rahmen der Messgenauigkeit des ¹H-NMR keinerlei Hinweise auf eine Reaktion der C=C Doppelbindung in der Mitte des Moleküls gefunden.

### Beispiel 7: Herstellung von Mevalonsäuredimethylamid

2,96 kg (20 mol) razemische 3,5-Dihydroxy-3-methylpentansäure (Mevalonsäure) wurden in einem 10 Liter Rührautoklaven (Büchi) unter Erwärmen auf 60 °C in 4 Litern Toluol dispergiert. Anschließend wurde die Säure langsam mittels Einleiten von 945 g gasförmigen Dimethylamins (21 mol) in das Ammoniumsalz überführt (Vorgehen entsprechend Beispiel 1). Nach Abklingen der Wärmetönung wurde das so erhaltene Ammoniumsalz bei einem Arbeitsdruck von etwa 25 bar kontinuierlich mit 4 l/h durch das Reaktionsrohr gepumpt und einer Mikrowellenleistung von durchschnittlich 2,25 kW ausgesetzt, von denen 88 % vom Reaktionsgut absorbiert wurden. Die Verweilzeit des Reaktionsgemischs in der Bestrahlungszone betrug ca. 42 Sekunden. Am Ende des Reaktionsrohres hatte das Reaktionsgemisch eine Temperatur von 250 °C.

Es wurde ein Umsatz von 92 % d. Th. erreicht. Das Reaktionsprodukt war gelblich rot gefärbt. In der Rohproduktmischung konnten weder Aminolyseprodukte noch Polymere nachgewiesen werden. Der Gehalt an Eisen betrug < 2 ppm. Das gewünschte Produkt 3,5-Dihydroxy-3-methylpentansäuredimethylamid wurde nach Abdampfen des Toluols und des entstandenen Reaktionswassers isoliert.

## Patentansprüche

1. Kontinuierliches Verfahren zur Herstellung von Hydroxycarbonsäureamiden, indem mindestens eine Hydroxycarbonsäure der Formel I
HO-R³-COOH (I)
worin R³ für einen gegebenenfalls substituierten aliphatischen Kohlenwasserstoffrest mit 1 bis 100 Kohlenstoffatomen steht,
mit mindestens einem Amin der Formel II
HNR¹R² (II)
worin R¹ und R² unabhängig voneinander für Wasserstoff oder einen Kohlenwasserstoffrest mit 1 bis 100 C-Atomen stehen, wobei R¹ oder R² oder beide Reste Substituenten ausgewählt aus C₁-C₅-Alkoxy-, Cyano-, Nitril-, Nitro- und C₅-C₂₀-Arylgruppen tragen können, und wobei falls R¹ oder R² oder beide Reste C₅-C₂₀-Arylgruppen tragen, diese einen oder mehrere Substituenten ausgewählt aus Halogenatomen, halogenierten Alkylresten, C₁-C₂₀-Alkyl-, C₂-C₂₀-Alkenyl-, C₁-C₅-Alkoxy-, Hydroxyl-, Ester-, Amid-, Cyano-, Nitril-, und Nitrogruppen tragen können,
oder worin R¹ und R² zusammen mit dem Stickstoffatom, an das sie gebunden sind, einen Ring bilden,
oder worin R¹ und/oder R² unabhängig voneinander für eine gegebenenfalls substituierte C₆-C₁₂-Arylgruppe oder eine gegebenenfalls substituierte heteroaromatische Gruppe mit 5 bis 12 Ringgliedern stehen,
oder worin R¹ und/oder R² unabhängig voneinander für einen mit Heteroatomen unterbrochenen Alkylrest stehen,
oder worin R¹ und/oder R² unabhängig voneinander für Reste der Formel III
-(R⁴-O)ₙ-R⁵ (III)
stehen, worin
R⁴ für eine Alkylengruppe mit 2 bis 6 C-Atomen,
R⁵ für einen Kohlenwasserstoffrest mit 1 bis 24 C-Atomen oder eine Gruppe der Formel NR¹⁰R¹¹,
n für eine Zahl zwischen 2 und 50 und
R¹⁰, R¹¹ unabhängig voneinander für einen aliphatischen Rest mit 1 bis 24 C-Atomen und bevorzugt 2 bis 18 C-Atomen, eine Arylgruppe- oder Heteroarylgruppe mit 5 bis 12 Ringgliedern, eine Poly(oxyalkylen)gruppe mit 1 bis 50 Poly(oxyalkylen)einheiten, wobei sich die Polyoxyalkyleneinheiten von Alkylenoxideinheiten mit 2 bis 6 C-Atomen ableiten, oder R¹⁰ und R¹¹gemeinsam mit dem Stickstoffatom, an das sie gebunden sind, einen Ring mit 4, 5, 6 oder mehr Ringgliedern bilden, stehen,
oder worin R¹ und/oder R² unabhängig voneinander für Reste der Formel IV
-[R⁶-N(R⁷)]ₘ-(R⁷) (IV)
stehen, worin
R⁶ für eine Alkylengruppe mit 2 bis 6 C-Atomen oder Mischungen daraus,
jedes R⁷ unabhängig voneinander für Wasserstoff, einen Alkyl- oder Hydroxyalkylrest mit bis zu 24 C-Atomen, einen Polyoxyalkylenrest -(R⁴-O)ₚ-R⁵, oder einen Polyiminoalkylenrest -[R⁶-N(R⁷)]_{q}-(R⁷), wobei R⁴, R⁵, R⁶ und R⁷ die oben angegebenen Bedeutungen haben und q und p unabhängig voneinander für 1 bis 50 stehen, und
m für eine Zahl von 1 bis 20 und bevorzugt 2 bis 10 wie beispielsweise drei, vier, fünf oder sechs stehen,
zu einem Ammoniumsalz umgesetzt wird und dieses Ammoniumsalz nachfolgend unter Mikrowellenbestrahlung in einem Reaktionsrohr, dessen Längsachse sich in der Ausbreitungsrichtung der Mikrowellen eines Monomode-Mikrowellenapplikators befindet, zum Hydroxycarbonsäureamid umgesetzt wird, und worin die Bestrahlung des Salzes mit Mikrowellen in einem weitgehend mikrowellentransparenten Reaktionsrohr innerhalb eines mit einem Mikrowellengenerator über Wellenleiter verbundenen Hohlleiters erfolgt.

2. Verfahren nach Anspruch 1, bei dem der Mikrowellenapplikator als Hohlraumresonator ausgestaltet ist.

3. Verfahren nach Anspruch 1 und/oder 2, bei dem der Mikrowellenapplikator als Hohlraumresonator vom Reflexionstyp ausgestaltet ist.

4. Verfahren nach einem oder mehreren der Ansprüche 1 bis 3, bei dem das Reaktionsrohr axial mit einer zentralen Symmetrieachse des Hohlleiters fluchtet.

5. Verfahren nach einem oder mehreren der Ansprüche 1 bis 4, bei dem die Bestrahlung des Salzes in einem Hohlraumresonator mit koaxialem Übergang der Mikrowellen erfolgt.

6. Verfahren nach einem oder mehreren der Ansprüche 1 bis 5, bei dem der Hohlraumresonator im E₀₁ₙ-Mode betrieben wird, wobei n eine ganze Zahl von 1 bis 200 ist.

7. Verfahren nach einem oder mehreren der Ansprüche 1 bis 6, bei dem R³ 1 bis 30 Kohlenstoffatome enthält.

8. Verfahren nach einem oder mehreren der Ansprüche 1 bis 7, bei dem R³ mindestens eine an ein sekundäres Kohlenstoffatom gebundene OH-Gruppe enthält.

9. Verfahren nach einem oder mehreren der Ansprüche 1 bis 8, bei dem R³ eine Hydroxylgruppe in α-Position zur Carboxylgruppe enthält.

10. Verfahren nach einem oder mehreren der Ansprüche 1 bis 9, bei dem R³ eine oder mehrere Doppelbindungen enthält.

11. Verfahren nach einem oder mehreren der Ansprüche 1 bis 10, bei dem R³ einen oder mehrere Substituenten ausgewählt aus Halogenatomen, halogenierten Alkylresten, C₁-C₅-Alkoxy-, Poly(C₁-C₅-Alkoxy)-, Poly(C₁-C₅-Alkoxy)alkyl-, Carboxyl-, Hydroxyl-, Ester-, Amid-, Cyano-, Nitril-, Nitro- und Arylgruppen mit 5 bis 20 Kohlenstoffatomen, wobei die C₅-C₂₀-Arylgruppen Substituenten ausgewählt aus Halogenatomen, C₁-C₂₀-Alkyl-, C₂-C₂₀-Alkenyl-, C₁-C₅-Alkoxy-, Ester-, Amid-, Hydroxy-, Cyano-, Nitril-, und/oder Nitrogruppen tragen können.

12. Verfahren nach einem oder mehreren der Ansprüche 1 bis 11, bei dem R¹ und R² unabhängig voneinander für einen Kohlenwasserstoffrest mit 1 bis 100 Kohlenstoffatomen stehen.

13. Verfahren nach einem oder mehreren der Ansprüche 1 bis 11, bei dem R¹ für einen Kohlenwasserstoffrest mit 1 bis 100 Kohlenstoffatomen und R² für Wasserstoff steht.

14. Verfahren nach einem oder mehreren der Ansprüche 1 bis 13, bei dem die Mikrowellenbestrahlung bei Temperaturen zwischen 150 und 500 °C durchgeführt wird.

15. Verfahren nach einem oder mehreren der Ansprüche 1 bis 14, bei dem die Mikrowellenbestrahlung bei Drücken oberhalb des Atmosphärendrucks erfolgt.

16. Verfahren nach einem oder mehreren der Ansprüche 1 bis 15, bei dem R¹ oder R² oder beide Substituenten unabhängig voneinander für einen aliphatischen Rest mit 1 bis 24 C-Atomen stehen.
